# DEMANDE DE BREVET EUROPEEN

(11) **EP 2 848 212 A1**
(43) Date de publication de la demande: **18.03.2015**
(21) Numéro de dépôt: 14184910.9
(22) Date de dépôt: 16.09.2014
(51) Int. Cl.: A61B 17/28, A61B 17/062, A61B 17/29

(54) **Dispositif de verrouillage pour une pince telle qu'un porte-aiguille**

(30) Priorité: 16.09.2013 FR 1358905
(71) Demandeur: Landanger, 52906 Chaumont Cedex 9 (FR)
(72) Inventeur: Landanger, Benoit, 21490 Ruffey Les Echirey (FR)
(74) Mandataire: Oudin, Stéphane

(57) **Abrégé**

La présente invention concerne une pince (50) comprenant deux branches (51, 52) montées ensemble par une liaison pivot (53), entre une position ouverte et une position fermée, et un dispositif de verrouillage/déverrouillage (100) apte à maintenir verrouillée ladite pince, ledit dispositif de verrouillage comprenant une pièce de verrouillage femelle (110) et une pièce de verrouillage mâle (120), caractérisée en ce que ladite pièce de verrouillage femelle (110) comprend en outre une butée (170) comprenant une surface formant came (180) apte à déplacer latéralement, dans ladite première direction, ledit ergot (130) lorsque ladite pince passe d'une position ouverte à une position fermée et apte à déplacer latéralement, dans la direction opposée à ladite première direction, ledit ergot (130) vers ladite cavité (160), ladite butée (171) évitant un retour intempestif de l'ergot (130) dans ladite direction opposée lorsque ladite pince passe d'une position ouverte à une position fermée.

## Description

### Domaine technique

La présente invention concerne un dispositif de verrouillage et notamment un dispositif de verrouillage pour une pince et préférentiellement un dispositif de verrouillage pour un porte-aiguille. La présente invention concerne également les pinces, porte-aiguille comprenant le dispositif de verrouillage selon l'invention.

### Technique antérieure

Lors des opérations de suture par couture, le chirurgien utilise des porte-aiguilles afin de faciliter la manipulation de l'aiguille utilisée.

Classiquement, le porte-aiguille se présente sous la forme d'une pince comprenant deux branches montées pivotantes l'une par rapport à l'autre. Chacune des deux branches comprend une section, d'un côté du pivot, faisant fonction de mors et une section, de l'autre côté du pivot, faisant fonction de poignée. Les branches pivotent entre une position ouverte et une position fermée où les deux mors sont placés en vis à vis et sont aptes à maintenir une aiguille.

Les poignées peuvent comprendre des ouvertures circulaires similaire à celles que l'on peut trouver sur des ciseaux ou être incurvées afin d'améliorer la préhension par le chirurgien.

La section faisant fonction de poignée est souvent de longueur beaucoup plus importante que la longueur de la section faisant fonction de mors. Cette différence de longueur permet d'augmenter la force appliquée sur l'aiguille et d'améliorer ainsi son maintien.

Par ailleurs, les porte-aiguilles de l'art antérieur comprennent souvent un dispositif de verrouillage permettant de maintenir les branches en position fermée. Ainsi, l'aiguille est parfaitement maintenue même lorsque la pression n'est plus exercée par l'utilisateur sur la poignée. Ce verrouillage est particulièrement important puisqu'il permet d'éviter la perte des aiguilles lors de l'opération.

Parmi les dispositifs de verrouillage connus de l'art antérieur, on peut citer les dispositifs consistant en deux lames comprenant chacune une face crantée venant s'associer par encliquetage lorsque les branches sont en position fermée. Chacune de ces deux lames est associée à une branche au niveau de la section formant poignée et s'étend perpendiculairement à ladite branche en direction de l'autre lame. Lors du passage de la position ouverte à la position fermée, les faces crantées des deux lames viennent en contact et les crantages interagissent avec les crantages de l'autre lame en vis-à-vis. Il arrive que l'opérateur puisse accidentellement déverrouiller ce type de dispositif en exerçant sur le porte-aiguille un mouvement de torsion provoquant la séparation des deux lames.

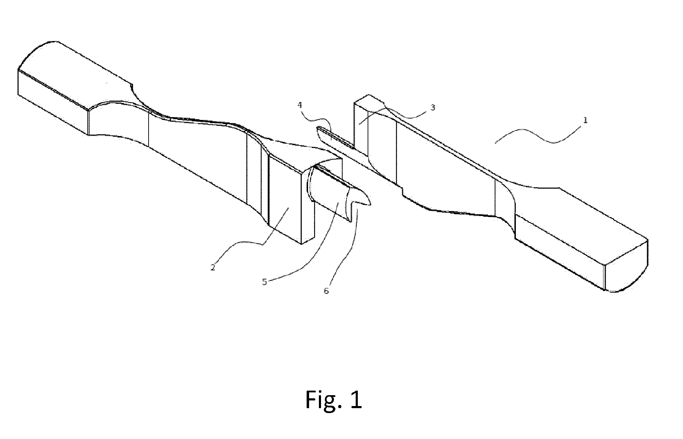

La figure 1 présente une vue en perspective d'un autre dispositif de verrouillage 1 connu de l'art antérieur. Ledit dispositif de verrouillage 1 comprend une pièce de verrouillage femelle 2 associée solidairement et disposée parallèlement à l'une des branches (non représentée) et une pièce de verrouillage mâle 3 associée solidairement et associée parallèlement à l'autre branche. La pièce de verrouillage mâle 3 comprend un ergot 4 et la pièce de verrouillage femelle 2 comprend une pièce formant came 5 comprenant une cavité 6, apte à recevoir ledit ergot 4 lorsque la pince est en position verrouillée. La pièce formant came 5 est apte à provoquer un mouvement de rotation de l'ergot 4 autour de la came 5 à chaque fois que les branches opèrent un mouvement d'ouverture-fermeture. Dans un premier temps, la came 5 va faire déplacer latéralement l'ergot 4 lorsque la pince passe de la position ouverte à la position fermée puis l'ergot 4 va venir se loger dans la cavité 6. Pour déverrouiller le dispositif, l'utilisateur doit augmenter la pression sur les poignées de la pince afin de faire sortir l'ergot 4 de la cavité 6, puis l'ergot 4 va glisser sur l'autre face de la came 5 pour retrouver sa position initiale.

Ce type de dispositif permet d'éviter la séparation des mors lorsqu'une torsion est appliquée sur le porte-aiguille. Toutefois, lorsque le praticien ferme totalement les mors, lors du passage de la position ouverte à la position fermée, l'ergot 4 peut ne pas se loger dans la cavité 6 et effectuer une rotation complète autour de la pièce formant came 5.

### Résumé de l'invention

La présente invention a pour but de répondre aux problèmes posés par les dispositifs de verrouillage de l'art antérieur en proposant un dispositif de verrouillage permettant d'éviter les défauts de verrouillage observés avec les dispositifs présentés à la figure 1.

Ainsi la présente invention concerne une pince comprenant deux branches montées ensemble par une liaison pivot, entre une position ouverte et une position fermée, et un dispositif de verrouillage/déverrouillage apte à maintenir verrouillée ladite pince, ledit dispositif de verrouillage comprenant une pièce de verrouillage femelle associée solidairement à l'une desdites deux branches et une pièce de verrouillage mâle associée à l'autre branche, ladite pièce de verrouillage mâle comprenant un ergot et ladite pièce de verrouillage femelle comprenant un cylindre comportant une cavité, apte à recevoir ledit ergot lorsque ladite pince est en position verrouillée, et , l'une desdites pièces de verrouillage femelle et mâle comprenant au moins un longeron faisant office de lame ressort et étant apte à faire déplacer latéralement dans une première direction ledit ergot relativement par rapport audit cylindre lorsque ladite pince passe de la position ouverte à la position fermée et à faire déplacer ledit ergot latéralement dans une direction opposée à ladite première direction lorsque ladite pince passe de ladite position fermée à ladite position ouverte remarquable en ce que ladite pièce de verrouillage femelle comprend en outre une butée comprenant une surface formant came apte à déplacer latéralement, dans ladite première direction, ledit ergot lorsque ladite pince passe d'une position ouverte à une position fermée et apte à déplacer latéralement, dans la direction opposée à ladite première direction, ledit ergot vers ladite cavité, ladite butée évitant un retour intempestif de l'ergot dans ladite direction opposée lorsque ladite pince passe d'une position ouverte à une position fermée.

Selon un mode de réalisation préféré de l'invention, ledit ergot est disposé sur la face interne de ladite branche.

Selon un mode de réalisation préféré de l'invention, ledit ergot est un cylindre dont l'axe longitudinal est sensiblement perpendiculaire à la section transversale de la branche, à laquelle il est associé.

Selon un mode de réalisation préféré de l'invention, ledit ergot a une section droite en forme d'ellipse dont l'axe principal forme un angle compris entre 50° et 60° avec le plan perpendiculaire à l'axe de ladite liaison pivot et comporte au moins une première et une deuxième faces convexes opposées et symétriques par rapport audit axe principal.

Selon un mode de réalisation préféré de l'invention, le cylindre de ladite pièce de verrouillage femelle est tel que son axe longitudinal est sensiblement perpendiculaire à la section transversale de la branche à laquelle il est associé et que sa surface externe forme un chemin de came recouvrant ladite cavité.

Selon un mode de réalisation préféré de l'invention, ledit chemin de came présente une section droite en forme globale de V inversé dont l'axe de symétrie est sensiblement parallèle à l'axe principal de l'ergot.

Selon un mode de réalisation préféré de l'invention, le chemin de came comporte deux faces convexes, une première face destinée à guider ledit ergot latéralement dans une première direction lorsque ladite pince passe de la position ouverte à la position fermée et une deuxième face destinée à guider ledit ergot lorsque ladite pince passe de ladite position fermée à ladite position ouverte.

Selon un mode de réalisation préféré de l'invention, ladite première face s'étend en direction de ladite butée.

Selon un mode de réalisation préféré de l'invention, la cavité présente une section droite en V inversée dont l'axe de symétrie est sensiblement parallèle à l'axe principal dudit ergot.

Selon un mode de réalisation préféré de l'invention, ladite butée forme une excroissance à la face interne de la branche à laquelle elle est associée.

Ainsi, dans le dispositif selon l'invention, lorsque la pince est en position fermée, l'ergot est maintenu latéralement par la butée qui va ensuite orienter ledit ergot vers ladite cavité lorsque l'utilisateur desserre légèrement la pression sur la pince. L'ergot ne peut donc plus, comme dans les dispositifs de verrouillage de l'art antérieur, effectuer une rotation complète autour de la came sans venir se placer dans la cavité.

### Brèves description des dessins

La figure 1 présente une vue en perspective d'un type de dispositif de verrouillage selon l'art antérieur.
La figure 2 présente une vue en perspective d'une pince munie d'un dispositif de verrouillage selon l'invention
La figure 3 présente une vue en perspective d'un mode de réalisation d'un dispositif de verrouillage selon l'invention
Les figures 4 10 présentent une vue en coupe transversale du dispositif de verrouillage présenté à la figure 2 dans les différentes phases d'utilisation.

### Description des modes de réalisation

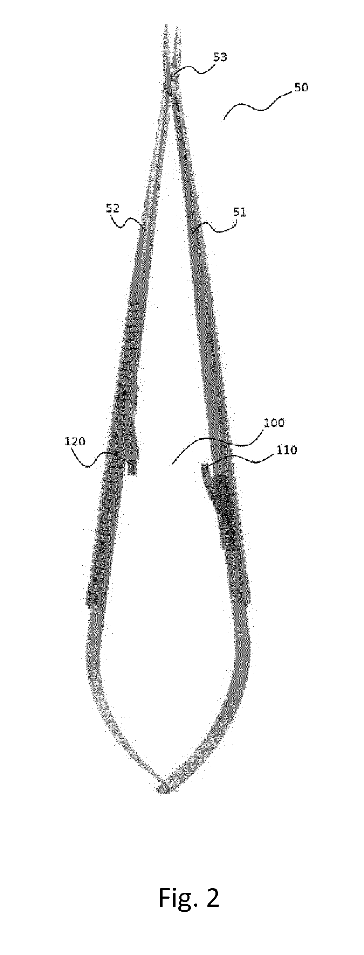

En référence à la figure 2, la pince 50 selon l'invention comprend deux branches 51, 52 montées ensemble par une liaison pivot 53, entre une position ouverte et une position fermée. La pince 50 selon l'invention comprend en outre un dispositif de verrouillage/déverrouillage 100 apte à maintenir verrouillée ladite pince. Le dispositif de verrouillage 100 comprend une pièce de verrouillage femelle 110 associée solidairement à l'une 51 desdites deux branches 51, 52 et une pièce de verrouillage mâle 120 associée à l'autre branche 52.

L'association entre les pièces de verrouillage mâle/femelle 110, 120 et les branches 51, 52 peut être réalisée de différentes façons. Lesdites pièces de verrouillage peuvent être moulées et/ou usinées avec lesdites branches. Elles peuvent être également moulées et/ou usinées séparément puis associées audites branches par tout moyen approprié (e.g. soudure, rivetage). Les pièces de verrouillage 110, 120 comprennent respectivement une face d'appui 111, 121 et préférentiellement un alésage 112, 122 apte à recevoir chacun un moyen de fixation (e.g. vis, rivet) pour associer solidairement lesdites pièces de verrouillage 110, 120 respectivement auxdites branches 51, 52 en mettant en contact les faces d'appui 111,121 et les faces internes desdites branches 51, 52.

La pièce de verrouillage mâle 120 comprend un ergot 130 et la pièce de verrouillage femelle 110 comprend un cylindre 150 comportant une cavité 160, apte à recevoir l'ergot 130 lorsque la pince 50 selon l'invention est en position verrouillée, la surface extérieure dudit cylindre 150 formant un chemin de came 151 entourant ladite cavité 160. La pièce de verrouillage femelle 110 comprend en outre une butée 170 dont une partie au moins de la une surface externe forme chemin de came 180.

Par « cylindre », on entend désigner le solide délimité par une surface cylindrique et par deux plans parallèles, ladite surface étant définie par une droite, appelée génératrice, passant par un point variable décrivant une courbe plane fermée, appelée courbe directrice et gardant une direction fixe.

La pièce de verrouillage mâle 120 et la pièce de verrouillage femelle 110 se trouve préférentiellement sur la face interne de la branche à laquelle ils sont respectivement associé. Par "face interne", on entend signifier que lesdites pièces de verrouillage 110, 120 sont disposées dans l'espace compris entre les deux branches 51, 52 de la pince 50 selon l'invention.

Lors du passage de la pince 50 selon l'invention, de la position ouverte à la position fermée, l'extrémité de l'ergot 130 la plus éloignée de la branche 52 à laquelle il est associé va parcourir un trajet initial compris dans un plan dit « plan initial ». Par « trajet initial », on entend désigner le trajet parcouru par ledit ergot 130 avant qu'il ne rentre en contact avec le chemin de came 151.

En plus des éléments nécessaires au verrouillage/déverrouillage de la pince 50 selon l'invention, les pièces de verrouillage 110, 120 comprennent respectivement un longeron 113, 123 comprenant au moins une face d'extrémité 114, 124 sensiblement perpendiculaire à la face d'appui 111, 121 et sur laquelle sont disposés l'ergot 130, la butée 170, et le cylindre 150. Chaque longeron 112, 123 comporte une portion médiane 115, 125 plus mince de sorte à faire office de lame ressort et à permettre un déplacement relatif des faces d'extrémités 114, 124 l'une par rapport à l'autre et un repositionnement de ces dernières dans une position initiale, correspondant à leur position lorsque la pince 50 est ouverte.

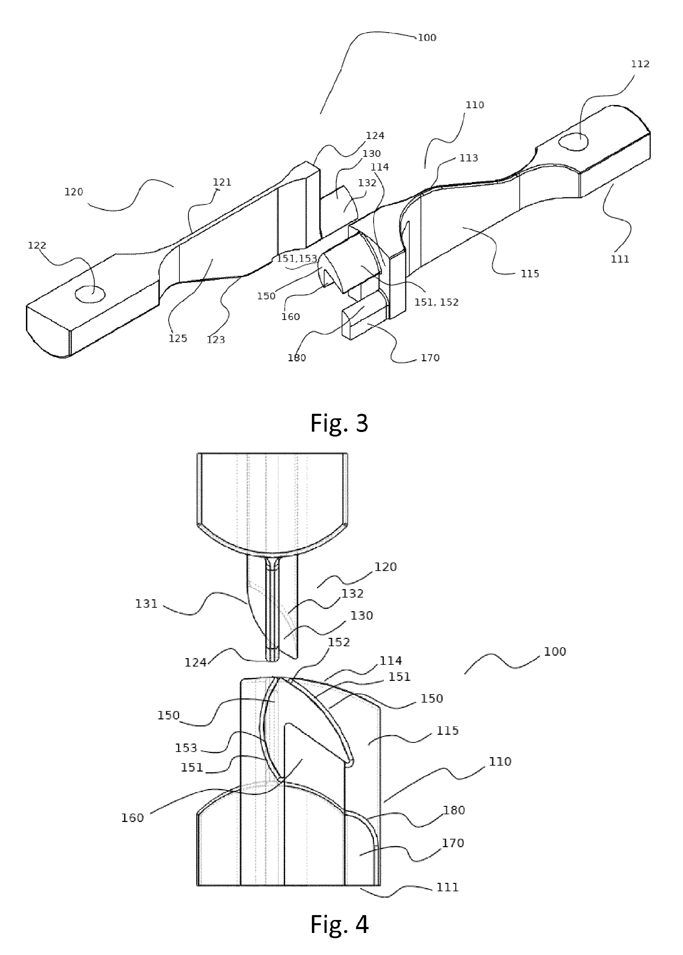

En référence à la figure 4, l'ergot 130 est un cylindre dont l'axe longitudinal est sensiblement perpendiculaire à la section transversale de la branche 52, à laquelle il est associé. Par "sensiblement perpendiculaire", on entend signifier que l'axe longitudinal de l'ergot 130 et la section transversale de la branche 52 à laquelle il est associé sont perpendiculaires ou forment un angle compris entre 85° et 95 ° et préférentiellement strictement parallèles.

L'ergot 130 a une section droite en forme globale d'ellipse dont l'axe principal forme un angle compris entre 45 et 90° avec le plan perpendiculaire à l'axe de ladite liaison pivot 53. Par « ellipse », on entend désigner les ellipses pures au sens mathématiques mais également les ovales à deux axes de symétrie. Ainsi, l'ergot 130 comporte au moins une première et une deuxième faces 131, 132 convexes opposées et symétriques par rapport audit axe principal, la première face 131 étant la plus éloignée de la branche 52 à laquelle elle est associée. L'axe longitudinal de l'ergot 130 est préférentiellement placé à une distance comprise entre 3.9 et 4.2 mm de la branche 52 à laquelle il est associé. Comme indiqué précédemment l'ergot 130 s'étend préférentiellement à partir de la face d'extrémité 124 du longeron 123. Il s'étend préférentiellement sur une longueur comprise entre 2.9 et 3.1 mm.

La pièce de verrouillage femelle 110 comprend donc un cylindre 150, dont l'axe longitudinal est sensiblement perpendiculaire à la section transversale de la branche 51 à laquelle il est associé. Le cylindre 150 comprend une cavité 160 et sa surface extérieure forme un chemin de came 151 entourant ladite cavité 160.Préférentiellement, ledit chemin de came 151présente une section droite en forme globale de V inversé dont l'axe de symétrie est sensiblement parallèle à l'axe principal de l'ergot 130. Ainsi, le chemin de came 151 comprend deux faces 152, 153. La première face 152 s'étend en direction de ladite butée 170. La deuxième face 153 s'étend préférentiellement sensiblement perpendiculairement à la face interne de la branche 51 à laquelle elle est associée. Encore plus préférentiellement, les deux faces 152, 153 présentent chacune, en section droite, un profil convexe.

La première face 152 coupe ledit plan initial afin que l'extrémité de l'ergot 130 la plus éloignée de la branche 52 avec laquelle il est associé rentre en contact avec la première face 152 du chemin de came 151 lorsque l'on resserre les branches 51, 52.

Préférentiellement, ladite cavité 160 présente une section droite en forme globale de V inversé dont l'axe de symétrie est sensiblement parallèle à l'axe principal de l' ergot 130.

La cavité 160 présente des dimensions compatibles avec celle de l'ergot 130.

La région située directement entre la cavité 160 et la face interne de la branche 52 de la pince 50, à laquelle la cavité 160 est associée, est avantageusement libre afin de permettre le déplacement de l'ergot 130 lors du déverrouillage de la pince 50.

La butée 170 forme préférentiellement une excroissance à la face interne de la branche 51 à laquelle elle est associée. Cette excroissance s'étend perpendiculairement à ladite face interne. Préférentiellement, la butée 170 présente une section droite en demi-arche dont la partie incurvée est intégrée à la surface formant chemin de came 180. Le sommet de ladite demi-arche est préférentiellement disposé à l'aplomb de l' extrémité inférieure de la face 152 du chemin de came 151.

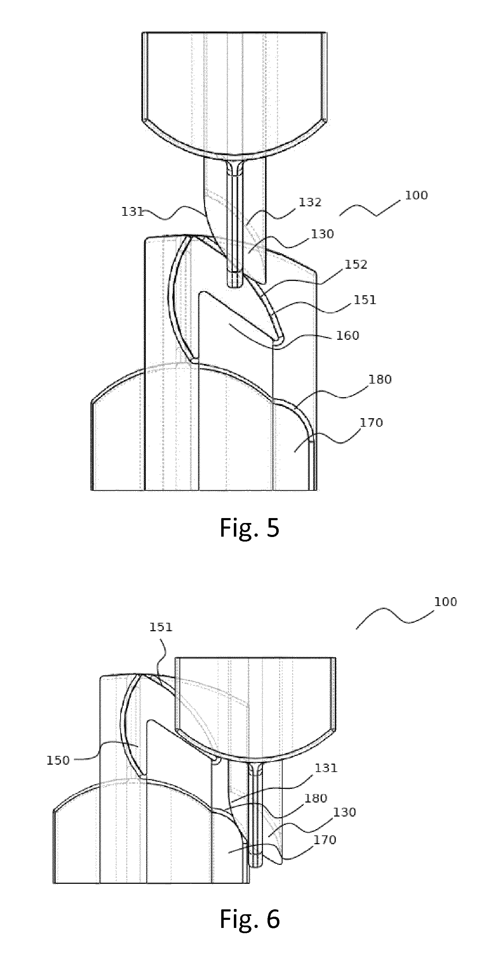

Les figures 4 à 10 présentent un cycle de verrouillage-déverrouillage d'une pince 50 selon l'invention. Lors du passage de la position ouverte à la position fermée, la première face 131 de l'ergot 130 vient au contact avec la première face 152 du chemin de came 151(figure 4). En augmentant la pression sur les poignées de la pince, l'utilisateur va entrainer le glissement de l'ergot 130 à la surface de la face 152 en direction de la butée 170 et donc grâce aux longerons 113, 123 un déplacement relatif des pièces de verrouillage mâle/femelle 110, 120 (figure 5). Lors de ce glissement, l'ergot 130 va se déplacer latéralement dans une première direction et rejoindre le chemin de came 180 de la butée 170 qui va également provoquer le déplacement latérale de l'ergot 130 dans ladite première direction (figure 6). Cette butée 170 permet d'éviter le retour intempestif de l'ergot 130 dans la direction opposée sous l'effet "ressort" des longerons 113, 123.

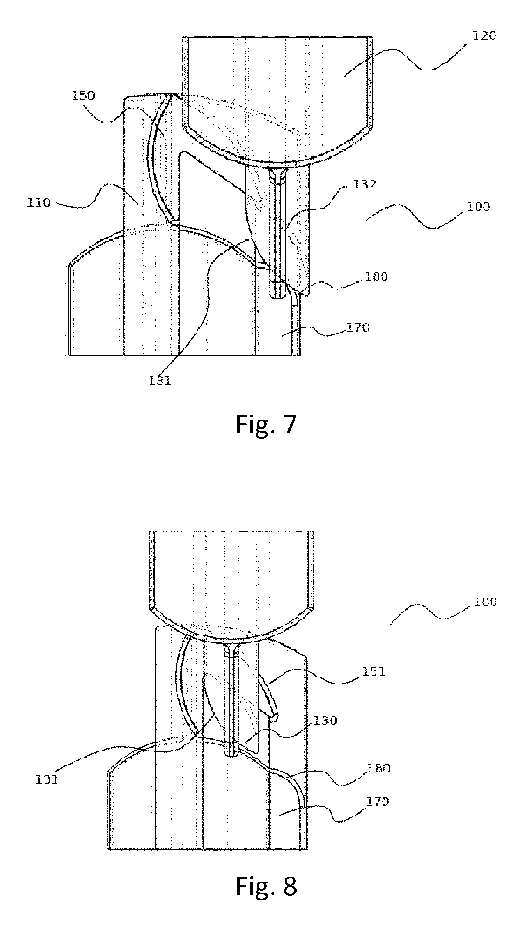

Lorsque l'utilisateur relâche la pression sur les poignées de la pince selon l'invention, le chemin de came 180 va diriger l'ergot 130 dans la cavité 160, sous l'effet "ressort" des longerons 113, 123, maintenant ainsi la pince 50 en position verrouillée (figures 7-8).

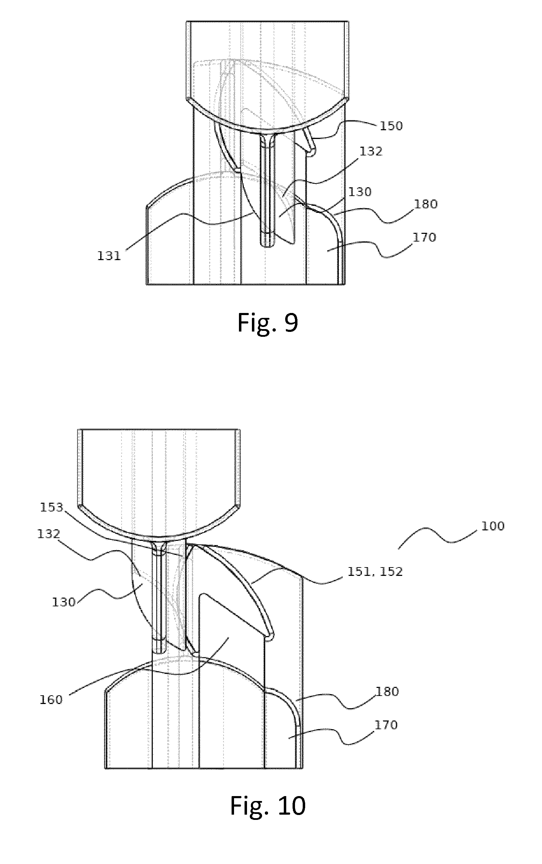

Une pression supplémentaire sur les branches 51, 52 de la pince 50 permet de déloger l'ergot 130 de la cavité 160 jusqu'à ce que, sous l'effet "ressort" des longerons 113, 123, la deuxième face 132 de l'ergot 130 rentre en contact avec la deuxième face 153 du chemin de came 151 du cylindre 150. Le relâchement de la pression sur les branches 51, va entrainer le déplacement latéral relatif de l'ergot 130 dans la direction opposée à la première direction (figures 9-10) et le retour de l'ergot 130 à sa position initiale, à savoir la position dans laquelle l'extrémité de l'ergot 130 la plus éloignée de la branche 52 à laquelle il est associé est dans le plan initial.

### Possibilité d'application industrielle

La pince 50 munie du dispositif de verrouillage/déverrouillage 100 selon l'invention s'applique plus particulièrement aux porte-aiguilles, mais elle peut également être utilisée pour d'autres instruments nécessitant le blocage sécurisé.

Enfin, il va de soi que les exemples de pince 50 munie du dispositif de verrouillage/déverrouillage 100 conformes à l'invention qui viennent d'être décrits ne sont que des illustrations particulières, en aucun cas limitatives de l'invention.

## Revendications

1. - Pince (50) comprenant deux branches (51, 52) montées ensemble par une liaison pivot (53), entre une position ouverte et une position fermée, et un dispositif de verrouillage/déverrouillage (100) apte à maintenir verrouillée ladite pince, ledit dispositif de verrouillage comprenant une pièce de verrouillage femelle (110) associée solidairement à l'une (51) desdites deux branches (51, 52) et une pièce de verrouillage mâle (120) associée à l'autre branche (52), ladite pièce de verrouillage mâle (120) comprenant un ergot (130) et ladite pièce de verrouillage femelle (110) comprenant un cylindre (150) comportant une cavité (160), apte à recevoir ledit ergot (130) lorsque ladite pince est en position verrouillée, et , les pièces de verrouillage femelle (110) et mâle (120) comprenant au moins un longeron (113, 123) faisant office de lame ressort et étant apte à faire déplacer latéralement dans une première direction ledit ergot (130) relativement par rapport audit cylindre (150) lorsque ladite pince passe de la position ouverte à la position fermée et à faire déplacer ledit ergot (130) latéralement dans une direction opposée à ladite première direction lorsque ladite pince passe de ladite position fermée à ladite position ouverte **caractérisée en ce que** ladite pièce de verrouillage femelle (110) comprend en outre une butée (170) comprenant une surface formant came (180) apte à déplacer latéralement, dans ladite première direction, ledit ergot (130) lorsque ladite pince passe d'une position ouverte à une position fermée et apte à déplacer latéralement, dans la direction opposée à ladite première direction, ledit ergot (130) vers ladite cavité (160), ladite butée (170) évitant un retour intempestif de l'ergot (130) dans ladite direction opposée lorsque ladite pince (50) passe d'une position ouverte à une position fermée.

2. - Pince (50) selon la revendication précédente **caractérisée en ce que** ledit ergot (130) est disposé sur la face interne de ladite branche.

3. - Pince (50) selon l'une des revendications précédentes **caractérisée en ce que** ledit ergot (130) est un cylindre dont l'axe longitudinal est sensiblement perpendiculaire à la section transversale de la branche (52), à laquelle il est associé.

4. - Pince (50) selon l'une des revendications précédentes **caractérisée en ce que** ledit ergot (130) a une section droite en forme d'ellipse dont l'axe principal forme un angle compris entre 50° et 60° avec le plan perpendiculaire à l'axe de ladite liaison pivot (53) et comporte au moins une première et une deuxième faces (131, 132) convexes opposées et symétriques par rapport audit axe principal.

5. - Pince (50) selon l'une des revendications précédentes **caractérisée en ce que** le cylindre (150) de ladite pièce de verrouillage femelle (110) est tel que son axe longitudinal est sensiblement perpendiculaire à la section transversale de la branche (51) à laquelle il est associé et que sa surface externe forme un chemin de came (151) recouvrant ladite cavité (160).

6. - Pince (50) selon la revendication précédente **caractérisée en ce que** ledit chemin de came (151) présente une section droite en forme globale de V inversé dont l'axe de symétrie est sensiblement parallèle à l'axe principal de l'ergot (130).

7. - Pince (50) selon la revendication précédente **caractérisée en ce que** le chemin de came (151) comporte deux faces (152, 153) convexes, une première face (152) destinée à guider ledit ergot (130) latéralement dans une première direction lorsque ladite pince (50) passe de la position ouverte à la position fermée et une deuxième face (153) destinée à guider ledit ergot (130) lorsque ladite pince (50) passe de ladite position fermée à ladite position ouverte.

8. - Pince (50) selon la revendication précédente **caractérisée en ce que** ladite première face (152) s'étend en direction de ladite butée (170).

9. - Pince (50) selon l'une des revendications précédentes **caractérisée en ce que** la cavité (160) présente une section droite en V inversée dont l'axe de symétrie est sensiblement parallèle à l'axe principal dudit ergot (130).

10. - Pince (50) selon l'une des revendications précédentes **caractérisée en ce que** ladite butée (170) forme une excroissance à la face interne de la branche à laquelle elle est associée.
